(19) Europäisches Patentamt
European Patent Office
Office européen des brevets

(11) **EP 3 228 612 B1**

(12) **EUROPEAN PATENT SPECIFICATION**

(45) Date of publication and mention
of the grant of the patent:
**03.07.2019  Bulletin 2019/27**

(51) Int Cl.:
***C07C 51/47*** *(2006.01)*     ***C07C 51/44*** *(2006.01)*
***C07C 57/07*** *(2006.01)*     ***C07C 57/04*** *(2006.01)*

(21) Application number: **15864552.3**

(86) International application number:
**PCT/JP2015/082681**

(22) Date of filing: **20.11.2015**

(87) International publication number:
**WO 2016/088578 (09.06.2016 Gazette 2016/23)**

(54) **METHOD FOR PURIFYING EASILY POLYMERIZABLE SUBSTANCE**

VERFAHREN ZUR REINIGUNG VON LEICHT POLYMERISIERBAREN SUBSTANZEN

PROCÉDÉ DE PURIFICATION DE SUBSTANCE FACILEMENT POLYMÉRISABLE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **03.12.2014  JP 2014245161**

(43) Date of publication of application:
**11.10.2017  Bulletin 2017/41**

(73) Proprietor: **Nippon Shokubai Co., Ltd.
Osaka-shi, Osaka 541-0043 (JP)**

(72) Inventors:
• **IMAZU, Hiroki
Himeji-shi
Hyogo 671-1292 (JP)**
• **MOTOFUJI, Masahiro
Himeji-shi
Hyogo 671-1292 (JP)**
• **KAKUTA, Hisao
Himeji-shi
Hyogo 671-1292 (JP)**
• **HISANAGA, Motoki
Himeji-shi
Hyogo 671-1292 (JP)**
• **HARA, Seiichi
Himeji-shi
Hyogo 671-1292 (JP)**

(74) Representative: **Müller-Boré & Partner
Patentanwälte PartG mbB
Friedenheimer Brücke 21
80639 München (DE)**

(56) References cited:
JP-A- 2001 129 388     JP-A- 2003 103 105
JP-A- 2003 103 161     JP-A- 2003 164 701
JP-A- 2007 217 403     JP-A- 2013 043 116
JP-A- 2013 203 712     US-A1- 2001 030 120
US-A1- 2002 008 064

**Description**

The present invention relates to a method for purifying an easily polymerizable substance, such as (meth)acrylic acid. More particularly, the present invention relates to a method comprising a separation step using a wet cyclone.

In production of an easily polymerizable substance, a separation/purification method by heating such as distillation is often adopted to separate the easily polymerizable substance from the impurities in a crude liquid containing the objective easily polymerizable substance and impurities. For example, US2001/030120 and US2002/008064 disclose methods for purifying acrylic acid using distillation. However, the easily polymerizable substances have a property of being polymerized by heating. Therefore, even after the separation of the impurities from the easily polymerizable substance, there is a case where the polymerized products may be left as insoluble solids in the liquid containing the easily polymerizable substance. As a means for removing insoluble solids, for example, as disclosed in Patent Document 1, there is a solid substance removing device comprising a body provided with a fluid inlet pipe and a fluid outlet pipe and a filtering part consisting of a metal mesh or the like disposed between the fluid inlet pipe and the fluid outlet pipe inside said body.

Patent Document 1: Japanese Patent Publication No. 2002-1017
Patent Document 2: Japanese Patent Publication No. 2001-293301

However, in the filtration method using a metal mesh disclosed in Patent Document 1, the metal mesh is likely to be clogged with the deposited insoluble solids, and therefore, it is necessary to clean the metal mesh regularly and frequently to eliminate clogging, which is troublesome. Most of easily polymerizable substances used in chemical plants are hazardous to the human body, and therefore, workers engaged in cleaning need to wear some protective gear, and they suffer much physical and mental stress when cleaning.

Under these circumstances, it is an object of the present invention to provide a method for purifying an easily polymerizable substance efficiently to improve the troublesome matters when cleaning, e.g., reducing the number of times the metal mesh is cleaned.

Conventionally, it has been understood that an easily polymerizable substance inevitably undergoes polymerization in a portion where a solution containing the easily polymerizable substance is retained (see paragraph 0003 in Patent Document 2, etc.). Therefore, according to conventional common knowledge in the art, if a solution containing an easily polymerizable substance is passed through a narrow pipe where insoluble solids easily clog, the easily polymerizable substance is likely to become retained in the pipe, and thus the easily polymerizable substance is feared to undergo polymerization at the retained portion. Further, a bottoms liquid immediately after being extracted from a distillation column has a high temperature. Thus, for example, if a bottoms liquid containing an easily polymerizable substance is handled in a high temperature state, a polymerized product is likely to be formed since the liquid is in a high temperature state. Therefore, apparatuses, devices, or the like having a narrow pipe diameter have not conventionally been used in a handling process of a hot bottoms liquid containing an easily polymerizable substance.

However, the present inventors have extensively studied to solve the above problems. As a result, contrary to the above conventional wisdom, the present inventors have found that a wet cyclone having a narrow pipe diameter may be used to separate insoluble solids contained in a hot bottoms liquid, and improve the troublesome matters caused by cleaning operations, to efficiently purify an easily polymerizable substance. Based on this finding, the present invention has been made.

That is, a method for purifying an easily polymerizable substance by distillation of the present invention is characterized by comprising:

a step of introducing a crude liquid containing the easily polymerizable substance into a distillation column,
a step of discharging a bottoms liquid from a collection part of the distillation column, and
a first separation step of introducing the bottoms liquid into a wet cyclone, to separate a first purified liquid containing the easily polymerizable substance from a liquid containing an insoluble solid,
wherein the easily polymerizable substance is an unsaturated carboxylic acid or an ester thereof. The insoluble solid contained in the bottoms liquid was separated by the wet cyclone, that makes it possible to remove the insoluble solid having a relatively large grain diameter efficiently. Thereby, the conventional problem about the clogging by the insoluble solid in the filtration may be solved, and a burden when cleaning may be reduced.

In a preferred embodiment, the insoluble solid having a grain diameter of not less than 450 $\mu$m is removed by the wet cyclone; and a ratio of removing the insoluble solid having a grain diameter of not less than 450 $\mu$m is not less than 90%.

In a preferred embodiment, a speed of introducing the bottoms liquid into the wet cyclone is not less than 0.1 m·s$^{-1}$ and not more than 2.0 m·s$^{-1}$.

In a preferred embodiment, the bottoms liquid introduced into the wet cyclone has a temperature of higher than 70°C and lower than 120°C.

Additionally, in a preferred embodiment, a flow rate per unit time of the liquid containing the insoluble solid is not more than 10% of that of the bottoms liquid.

Furthermore, in a preferred embodiment, the present invention further comprising:
a second separation step of separating at least a portion of the insoluble solid comprised in the liquid containing the insoluble solid to obtain a second purified liquid containing the easily polymerizable substance. The liquid containing the insoluble solid separated by the wet cyclone is further subjected to a second separation step, thereby the easily polymerizable substance remaining in the liquid containing the insoluble solid may be efficiently collected.

Furthermore, in a preferred embodiment, the present invention comprising:
a returning step of introducing the second purified liquid containing the easily polymerizable substance into the distillation column. That makes it possible to collect the easily polymerizable substance remaining in the second purified liquid containing the easily polymerizable substance more efficiently.

Furthermore, in a preferred embodiment of the present invention, the easily polymerizable substance is acrylic acid, methacrylic acid, an acrylic acid ester, or a·methacrylic acid ester.

According to the present invention, the bottoms liquid discharged (or extracted) from the collection part of the distillation column is introduced into the wet cyclone so that the first purified liquid containing the easily polymerizable substance is separated from the liquid containing the insoluble solid, whereby the number of times the separation device is cleaned may be reduced, and therefore, it is possible to purify the easily polymerizable substance efficiently. In addition, the present method may allow a worker to be less often exposed to chemical substances during cleaning. Thus, the present method is preferable for the worker in terms of mental health and safety. Furthermore, after the second separation step, the second purified liquid containing the easily polymerizable substance is introduced and returned into the distillation column, whereby the loss of the easily polymerizable substance may be reduced, and therefore, it is possible to improve the production efficiency of the easily polymerizable substance.

  FIG. 1 is a schematic drawing showing a flow of a method for purifying an easily polymerizable substance according to the present invention.
  FIG. 2A is a schematic drawing showing a front view of a wet cyclone used in the examples.
  FIG. 2B is a schematic drawing showing a side view of the wet cyclone used in the examples.

A method for purifying an easily polymerizable substance according to the present invention is characterized by comprising a step of introducing a crude liquid containing the easily polymerizable substance into a distillation column, and a first separation step of introducing a bottoms liquid extracted from a collection part of the distillation column into a wet cyclone to separate a first purified liquid containing the easily polymerizable substance from a liquid containing an insoluble solid. Wet cyclones do not have a mesh for collecting solid matter that a strainer or the like has. Thus, the wet cyclones will not be clogged even when the wet cyclone runs for a long time. Therefore, although it took much time and cost to clean the strainer and also put a burden on a worker conventionally, these problems may be solved by employing a wet cyclone. In addition, the use of a wet cyclone allows separation of an insoluble solid having a relatively large grain diameter at a high removal ratio. Note that even when a wet cyclone is employed, a ratio of removing an insoluble solid having a relatively large grain diameter is similar to that of a strainer. In the description that follows, the method for purifying an easily polymerizable substance according to the present invention will be specifically described with reference to the accompanying drawings. The present invention is not intended to be limited to examples shown in the drawings. The present invention may be carried out with appropriate modifications within the range adaptable to the present invention, and such modifications are included in the technical scope of the present invention.

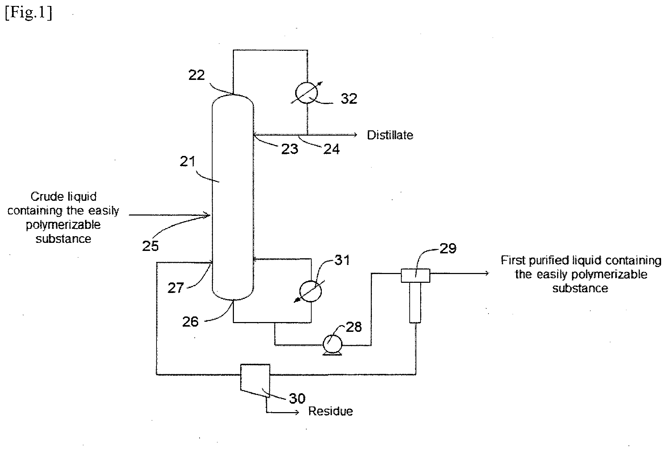

FIG. 1 shows a flow of the method for purifying an easily polymerizable substance according to the present invention.

In the present disclosure, the term "easily polymerizable substance" refers to a polymerizable monomer which may be polymerized by temperature, pressure, contact, agitation, or the like, and includes unsaturated carboxylic acids such as acrylic acid, methacrylic acid, fumaric acid, maleic acid, maleic anhydride; and esters thereof.

Examples of a hydroxy group-containing compound that may form esters with the above unsaturated carboxylic acids include aliphatic alcohols or alicyclic alcohols having 1 to 12 carbon atoms. Examples of the hydroxy group-containing compound include: monohydric alcohols such as methanol, ethanol, n-butanol, isobutanol, sec-butanol, t-butanol, 1-pentanol, 2-pentanol, 3-pentanol, cyclopentanol, 1-hexanol, 2-hexanol, 3-hexanol, cyclohexanol, 1-heptanol, 2-heptanol, 3-heptanol, 1-octanol, isooctanol, 2-ethylhexanol, isononyl alcohol, lauryl alcohol; polyhydric alcohols such as ethylene glycol, 1,3-propanediol; and the like. These may have a straight-chain or a branched-chain. These may be used singly or in a combination of two or more.

The easily polymerizable substance is preferably acrylic acid, methacrylic acid, an acrylic acid ester, or a methacrylic acid ester, more preferably acrylic acid, methacrylic acid, methyl acrylate, ethyl acrylate, butyl acrylate, 2-ethylhexyl acrylate, 2-hydroxyethyl acrylate, 2-hydroxypropyl acrylate, methyl methacrylate, ethyl methacrylate, butyl methacrylate, 2-ethylhexyl methacrylate, 2-hydroxyethyl methacrylate, or 2-hydroxypropyl methacrylate, and even more preferably acrylic acid or methacrylic acid.

In the present disclosure, the term "crude liquid containing an easily polymerizable substance" refers to a liquid which comprises the easily polymerizable substance and impurities, and may be introduced into a distillation column. Examples of the crude liquid containing an easily polymerizable substance include: a (meth)acrylic acid-containing liquid obtained by condensation of a (meth)acrylic acid-containing gas produced by a catalytic gas phase oxidation; a (meth)acrylic acid-containing liquid which is obtained by collection of a (meth)acrylic acid-containing gas produced by a a catalytic gas phase oxidation with a solvent (e.g., water, organic acid-containing water, or an inert hydrophobic organic liquid having a high boiling point (e.g., diphenyl ether, diphenyl, etc.)); partially purified (meth)acrylic acid which is obtained by purification of these (meth)acrylic acid-containing liquids; and the like. More specifically, it is a column bottom flow, a column side flow or the like extracted from such as a collection column, a condensation column, or a distillation column; or a liquid containing an easily polymerizable substance obtained by purification of the column bottom flow or the column side flow using a distillation step and/or crystallization step. When the easily polymerizable substances are an acrylic acid and an acrylic acid ester, the crude liquid containing the easily polymerizable substance is allowed to comprise an acrylic acid, by-products produced by a catalytic gas phase oxidation (e.g., water, an acetic acid, a maleic acid, aldehydes, a polymer of acrylic acid, etc.), and a solvent for collection of the acrylic acid-containing gas. When the easily polymerizable substances are a methacrylic acid and a methacrylic acid ester, the crude liquid containing the easily polymerizable substance is allowed to comprise a methacrylic acid, by-products produced by a catalytic gas phase oxidation (e.g., methacrolein, acetic acid, etc.), a solvent for collection of the methacrylic acid-containing gas.

The crude liquid containing an easily polymerizable substance may comprise a polymerization inhibitor. Examples of the polymerization inhibitor preferably include:

phenol compounds such as hydroquinone, methoxyhydroquinone, methoquinone, cresol, phenol, t-butylcatechol;
aromatic amines such as diphenylamine, p-phenylenediamine;
phenothiazine compounds such as phenothiazine, methylene blue;
copper salt compounds such as copper dialkyldithiocarbamates (e.g., copper dimethyldithiocarbamate, copper diethyldithiocarbamate, and copper dibutyldithiocarbamate), copper salicylate, copper acetate, copper naphthenate, copper acrylate, copper sulfate, copper nitrate, copper chloride;
manganese salt compounds such as manganese acetate;
N-oxyl compounds such as 4-hydroxy-2,2,6,6-tetramethylpiperidine-1-oxyl;
nitroso compounds such as N-nitrosophenylhydroxyl amine and ammonium salts thereof, p-nitrosophenol, N-nitrosodiphenylamine;
piperidine compounds such as N-hydroxy-2,2,6,6-tetramethylpiperidine, 2,2,6,6-tetramethylpiperidine;
ureas such as urea, urea derivatives;
thioureas such as thiourea, thiourea derivatives; and the like.

These compounds may be used singly or in a combination of two or more. The method of adding the polymerization inhibitors is not particularly limited. The polymerization inhibitors, for example, may be added directly in the form of such as solid or powder, or may be added in the form of a solution dissolved in a suitable solvent such as (meth)acrylic acid, a (meth)acrylic acid ester, water, an organic solvent, or the like. The polymerization inhibitors may also be dissolved in a process liquid in each step such as a material liquid, reflux liquid, absorbent liquid, or the like.

A column top part of a "distillation column" 21 has an "outlet of impurities having a low boiling point" 22, and an "inlet of reflux liquid" 23. A column bottom part of the "distillation column" 21 has an "outlet of the bottoms liquid" 26 for discharging a bottoms liquid. An "inlet of a treated liquid" 25 is provided at any suitable position between the column top part and the column bottom part, which is connected to an introducing pipe for feeding a crude liquid containing an easily polymerizable substance. The "inlet of reflux liquid" 23 is connected to an "introducing pipe of reflux liquid" 24. In the present invention, it is preferable that a "returning inlet" 27 is provided for returning a second purified liquid containing the easily polymerizable substance.

The distillation column may be a single distillation column, a packed column, a bubble-cap column, or a perforated-plate column. In particular, it is preferable that a distillation column having a plurality of sieve trays, or a distillation column having packed substances. A multiple perforated-plate column is preferable in terms of prevention of forming a polymer.

A reboiler 31 is used in the distillation column. The type of the reboiler is not particularly limited. When a multitubular heat exchanger is used as the reboiler, a vertical multitubular thermosiphon reboiler, a vertical shell-and-tube falling liquid film reboiler, or a forced circulation reboiler may be used.

Impurities having a low boiling point are discharged from the column top part of the distillation column. The term "impurities having a low boiling point" refers to impurities having a boiling point lower than that of the easily polymerizable substance. Examples of the impurities having a low boiling point include water, acetic acid, acrolein, or methacrolein.

Meanwhile, a bottoms liquid, from which all or a portion of impurities having a low boiling point have been removed, is extracted from a collection part of the distillation column. In the present disclosure, the term "collection part" refers to a part of the distillation column that is located below the inlet of the treated liquid, where the introducing pipe for feeding the crude liquid containing the easily polymerizable substance is connected. Therefore, in the distillation column 21 of FIG. 1, a part below the inlet of the treated liquid 25, where the introducing pipe for feeding the crude liquid containing the easily polymerizable substance is connected, corresponds to the collection part. In the present disclosure, the term "bottoms liquid" refers to a liquid discharged from the column bottom part of the distillation column. The bottoms liquid comprises an insoluble solid such as a polymer of the easily polymerizable substance, and other components such as the easily polymerizable substance, high boiling point components contained in the crude liquid containing the easily polymerizable substance. Therefore, in FIG. 1, a liquid extracted from the outlet of the bottoms liquid 26, which is connected to the collection part of the distillation column 21, corresponds to a bottoms liquid.

In the present disclosure, the term "insoluble solid" refers to a solid matter which is caused by impurities, and/or a polymer which is formed by polymerization of the easily polymerizable substance, which are contained in the crude liquid containing the easily polymerizable substance; and the insoluble solid is insoluble in the bottoms liquid. The polymer having a low degree of polymerization may have a property of being dissolved in the bottoms liquid. Meanwhile, the polymer having a high degree of polymerization has the property of being insoluble in the bottoms liquid. Insoluble solids may include insoluble components such as carbides produced by carbonization of the above polymers of the easily polymerizable substance by heat or the like. The bottoms liquid extracted from the distillation column is transported to a first separation step described below.

1. First Separation Step

In the first separation step, the bottoms liquid extracted from the collection part of the distillation column 21 is introduced into a wet cyclone 29, to separate a first purified liquid containing the easily polymerizable substance from a liquid containing an insoluble solid. The insoluble solid, such as a polymer of the easily polymerizable substance or the like, is contained in the bottoms liquid discharged from the collection part of the distillation column. In the present invention, the wet cyclone is used to separate the insoluble solid from the extracted bottoms liquid, and therefore, unlike the conventional art, the insoluble solid may be separated without passing the bottoms liquid through a filter, whereby cost and burden required for cleaning may be reduced.

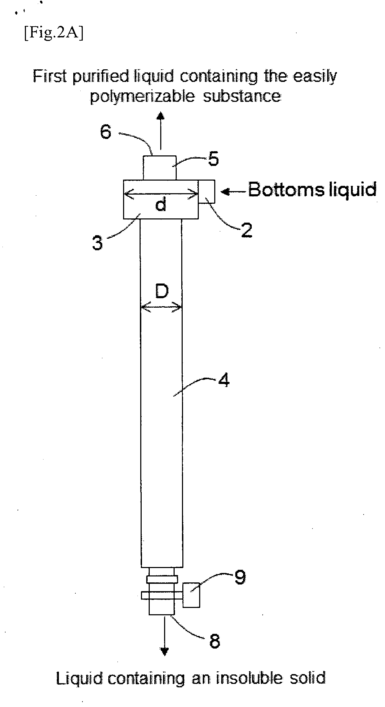

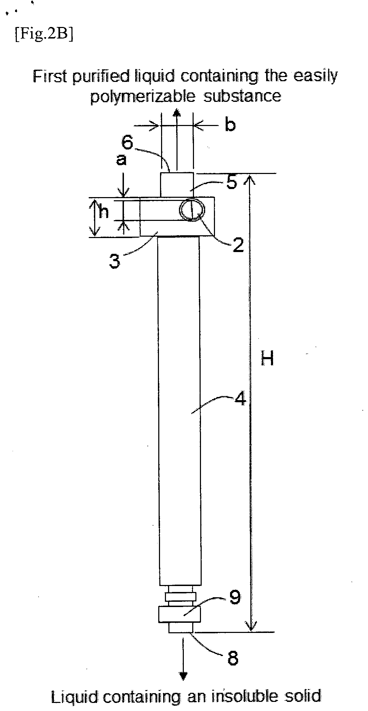

In the present disclosure, the term "wet cyclone" refers to a classifier that separates solid particles from a liquid containing the solid particles which are suspended (also hereinafter referred to as a "particles-suspended liquid"), by centrifugal force. FIG. 2A and FIG. 2B are schematic drawings of a representative example of the wet cyclone which is preferably used in the present invention. Note that the wet cyclone which may be used in the present invention is not limited to the type shown.

The wet cyclone comprises: an introduction part 2 for introducing a particles-suspended liquid; a cylindrical inlet/outlet part 3; a hopper part 4 provided below and adjacent to the inlet/outlet part 3; and a discharge part 5 provided above and adjacent to the inlet/outlet part 3. In the present invention, the particles-suspended liquid is particularly the bottoms liquid. The bottoms liquid is introduced tangentially into the inlet/outlet part 3 through the introduction part 2, and creates a vortex at a high speed in the cylindrical inlet/outlet part 3. An insoluble solid such as a polymer having a large specific gravity collides with a wall surface of the inlet/outlet part 3 by centrifugal force, thereafter, falls down in the direction of gravity in the hopper part 4, and is separated as a liquid containing the insoluble solid in a lower portion of the wet cyclone. Note that, in the present disclosure, the term "liquid containing an insoluble solid" refers to a liquid which is separated by a wet cyclone and comprises an insoluble solid having a large grain diameter at a high concentration. Although described in detail below, the liquid containing an insoluble solid preferably comprises most of insoluble solids comprised in the bottoms liquid. In particular, the liquid containing an insoluble solid preferably comprises the insoluble solid having a grain diameter of not less than 450 $\mu$m at a high concentration.

Meanwhile, an upward flow occurs at a center portion of the inlet/outlet part 3, and therefore, an insoluble solid such as a polymer having a relatively small grain diameter and a small specific gravity is carried by the upward flow and moved to the discharge part 5. Thus, by using the wet cyclone, an insoluble solid such as a polymer may be separated depending on the specific gravity by centrifugal force. As the grain diameter of an insoluble solid, particularly a polymer, increases, the mass of the polymer increases. Therefore, a polymer having a relatively large grain diameter is collected in the liquid containing an insoluble solid.

The discharge part 5 of the wet cyclone has an upper exit 6. An upper discharging pipe (not shown) is connected to the upper exit 6. The hopper part 4 of the wet cyclone has a lower exit 8. A lower discharging pipe (not shown) is connected to the lower exit 8. The first purified liquid containing the easily polymerizable substance obtained after the first separation step is discharged from the upper exit 6. The liquid containing an insoluble solid is discharged out of the wet cyclone through the lower exit 8. When the liquid containing an insoluble solid is discharged from the lower exit 8, a valve 9 provided below the hopper part 4 may be opened. Note that, in the present disclosure, the term "first purified liquid containing an easily polymerizable substance" refers to a liquid which is separated by a wet cyclone, and is obtained from the upper exit of the wet cyclone. Note that the first purified liquid containing an easily polymerizable substance may comprise an insoluble solid at a low concentration.

The introduction part 2 is preferably a cylindrical pipe. Specifically, the pipe diameter "a" of the introduction part 2 is preferably not less than 40 mm, more preferably not less than 45 mm, preferably not more than 100 mm, more preferably not more than 80 mm. Note that, in the present disclosure, the term "pipe diameter" means an inside diameter and a diameter of the pipe.

The inlet/outlet part 3 is also preferably a cylindrical pipe. The pipe diameter "d" of the inlet/outlet part 3 is preferably not less than 120 mm, more preferably not less than 150 mm, preferably not more than 300 mm, more preferably not more than 250 mm. The length "h" of the inlet/outlet part 3 is preferably not less than 50 mm, more preferably not less than 70 mm, preferably not more than 150 mm, more preferably not more than 120 mm.

In FIG. 2A and FIG. 2B, the hopper part 4 is cylindrical. The shape of the hopper part 4 is not limited to this, and may be, for example, conical. The hopper part 4 may have two or more stages. When the hopper part 4 has a two-stage configuration, the hopper part 4 may have an upper hopper part adjacent to the inlet/outlet part 3, and a lower hopper part adjacent to the lower exit 8. The upper hopper part may be cylindrical, and the lower hopper part may be conical. Furthermore, the hopper part 4 may have three stages. When the hopper part 4 has a three-stage configuration, the hopper part 4 may have an upper hopper part adjacent to the inlet/outlet part 3, a lower hopper part adjacent to the lower exit 8, and a middle hopper part between the upper hopper part and the lower hopper part. The upper hopper part and the middle hopper part may be cylindrical, and the lower hopper part may be conical.

The hopper part 4 preferably has a length of, for example, not less than 800 mm, more preferably not less than 820 mm, preferably not more than 1500 mm, more preferably not more than 1200 mm. The length of the hopper part 4 is preferably not less than two times, more preferably not less than five times, preferably not more than 12 times, more preferably not more than 10 times as great as the length "h" of the inlet/outlet part 3.

The pipe diameter of the hopper part 4 is not particularly limited. The maximum diameter is preferably not less than 50 mm, more preferably not less than 70 mm, preferably not more than 300 mm, more preferably not more than 200 mm in case where the hopper part 4 is cylindrical or conical. The maximum diameter of the hopper part 4 is preferably not less than 0.3 times, more preferably not less than 0.4 times, preferably not more than 0.9 times, more preferably not more than 0.7 times as great as the pipe diameter "d" of the inlet/outlet part 3.

When the hopper part 4 is conical, the pipe diameter of the hopper part 4 preferably becomes narrower from the inlet/outlet part 3 toward the lower exit 8. The pipe diameter of a bottom portion of the hopper part 4 is preferably, for example, not less than 20 mm and not more than 200 mm.

The upper exit 6 of the discharge part 5 preferably has a pipe diameter "b" of not less than 40 mm, more preferably not less than 50 mm, preferably not more than 120 mm, more preferably not more than 100 mm.

The wet cyclone preferably has a length "H" of not less than 900 mm, more preferably not less than 1000 mm, preferably not more than 1800 mm, more preferably not more than 1700 mm. The length "H" of the wet cyclone is preferably not less than two times, more preferably not less than seven times, preferably not more than 15 times, more preferably not more than 13 times as great as the length "h" of the inlet/outlet part 3.

An insoluble solid may be efficiently removed from the bottoms liquid by processing the bottoms liquid with the wet cyclone. For separating fine insoluble solids more than necessary, it is necessary to make the shape of the wet cyclone narrower to increase the speed of introducing the bottoms liquid. However, if the wet cyclone is made narrower, the inside of the wet cyclone is likely to be clogged with an insoluble solid and the easily polymerizable substance will be retained. Therefore, polymerization will likely occur at a portion where the easily polymerizable substance is retained. Meanwhile, if the first purified liquid containing the easily polymerizable substance comprises a large quantity of an insoluble solid having a grain diameter of not less than 450 $\mu$m, in the following purification step or the like, the insoluble solid will additionally generate polymer, or will trigger a clogging of pipes and devices. Therefore, it is desirable that, in the wet cyclone, an insoluble solid having a relatively large grain diameter should be separated as a liquid containing the insoluble solid, and thereby the insoluble solid may be efficiently removed while inhibiting an additional generation of an insoluble solid. For such a reason, it is preferable that the wet cyclone should separate an insoluble solid having a grain diameter of not less than 450 $\mu$m, more preferably an insoluble solid having a grain diameter of not less than 350 $\mu$m. A ratio of removing an insoluble solid having a grain diameter of not less than 450 $\mu$m is preferably, for example, not less than 90%, more preferably not less than 95%, and even more preferably not less than 98%. Note that, in the present disclosure, the term "a ratio of removing an insoluble solid having a grain diameter of not less than 450 $\mu$m" refers to "the total weight of an insoluble solid having a grain diameter of not less than 450 pm in the liquid containing an insoluble solid"/"the total weight of an insoluble solid having a grain diameter of not less than 450 $\mu$m in the bottoms liquid."

Note that the grain diameter of an insoluble solid may be measured with, for example, a laser diffraction grain size distribution analyzer.

The speed of introducing the bottoms liquid into the wet cyclone is preferably not less than 0.1 m ·s$^{-1}$, more preferably not less than 0.3 m ·s$^{-1}$, preferably not more than 2.0 m ·s$^{-1}$, more preferably not more than 1.5 m ·s$^{-1}$ in terms of efficiency of separation.

The pressure loss in the wet cyclone decreases with an increase in the area of a cross-section of the introduction part 2, and increases with an increase of the length "H" in the wet cyclone. In the present invention, the pressure loss in the wet cyclone is preferably 1 kPa to 50 kPa.

The wet cyclone may be provided to a pipe in a direction in which the bottoms liquid flows. The temperature of the bottoms liquid collected from the collection part of the distillation column is, for example, higher than 70°C and lower than 120°C, preferably not lower than 80°C and not higher than 110°C. The bottoms liquid collected from the collection part of the distillation column may be temporarily transported to a storage device such as a tank before being introduced into the wet cyclone. In the storage device, cooling is not generally performed, and therefore, the temperature of the bottoms liquid introduced into the wet cyclone is similar in temperature of the bottoms liquid collected by the distillation column, i.e. higher than 70°C and lower than 120°C, preferably not lower than 80°C and not higher than 110°C. To introduce the bottoms liquid into the wet cyclone, a liquid sending pump 28 is preferably provided to a pipe connecting the distillation column and the wet cyclone.

The liquid containing an insoluble solid may be continuously discharged through the lower discharge pipe of the wet cyclone, or may be intermittently discharged by opening and closing a valve provided in the lower discharge pipe of the wet cyclone. In the latter case, the easily polymerizable substance possibly undergoes a polymerization due to the insoluble solid that is temporarily retained in a lower portion of the wet cyclone. In some cases, it is necessary to stop the device for cleaning. Thereby, that would lead to an increase in loss of acrylic acid and a decrease in purification efficiency. Therefore, in the present invention, the former embodiment is preferable where the liquid containing an insoluble solid is continuously discharged through the lower discharge pipe of the wet cyclone. In this case, a flow rate per unit time of the liquid containing an insoluble solid, which is discharged through the lower discharge pipe, is preferably not less than 0.1%, more preferably not less than 1%, and even more preferably not less than 2%, preferably not more than 10%, more preferably not more than 5% of that of the bottoms liquid.

Note that the easily polymerizable substance in the first purified liquid containing the easily polymerizable substance may subsequently be further purified by an operation such as distillation to produce a product.

2. Second Separation Step

The present invention preferably comprises, after the first separation step, a second separation step of separating at least a portion of an insoluble solid, such as a polymer of the easily polymerizable substance, comprised in the liquid containing an insoluble solid, to obtain a second purified liquid containing the easily polymerizable substance. By removing the insoluble solid comprised in the liquid containing the insoluble solid, the easily polymerizable substance remaining in the liquid containing the insoluble solid may be efficiently collected.

The method for separating at least a portion of an insoluble solid, such as a polymer, from the liquid containing the insoluble solid in the second separation step is not particularly limited as long as the method may separate the solids from the liquid. The method includes a solid-liquid separation means 30 such as a gravitational sedimentation, a filtration, or a cyclonic separation. These may be used in combination. Of these methods, a gravitational sedimentation is particularly preferable for the second separation step because the operating cost is low, the filter is unlikely to be clogged by the insoluble solid, and the frequency of a cleaning operation is low.

When the gravitational sedimentation is adopted, a gravitational sedimentation tank may be used. The gravitational sedimentation tank preferably has a volume of not less than 30 L, more preferably not less than 40 L, preferably not more than 100 L, more preferably not more than 80 L. The gravitational sedimentation tank preferably has a horizontally projected area of not less than 0.03 m$^2$, more preferably not less than 0.05 m$^2$, preferably not more than 0.15 m$^2$, more preferably not more than 0.1 m$^2$.

If the liquid containing an insoluble solid is retained in the gravitational sedimentation tank for a long period of time, the easily polymerizable substance is likely to undergo a polymerization in the gravitational sedimentation tank. Therefore, for preventing polymerization, the retention time of the liquid containing an insoluble solid is preferably not longer than 48 hours, more preferably not longer than 20 hours. For a sufficient separation of the liquid and the solid, the retention time of the liquid containing the insoluble solid is preferably not shorter than 5 hours, more preferably not shorter than 10 hours. If the retention time is excessively short, the insoluble solid is unlikely to sink sufficiently, leading to a decrease in the efficiency of separation of the insoluble solid.

In the filtration, a strainer, a pall filter, a metal mesh, or the like may be used. The filtration is preferable because only insoluble solids having a large diameter may be removed, and most of the liquid may be collected. In addition, the quantity of the liquid containing an insoluble solid is smaller than the quantity of the bottoms liquid. Thus, even if the filtration is adopted, it is possible to make the size of the used metal mesh smaller. Therefore, compared to when the bottoms liquid in itself is subjected to filtration with the metal mesh or the like, the time and the burden for cleaning and the loss of the easily polymerizable substance may be reduced.

The filter used in the filtration preferably has a net-like structure. The net-like structure preferably has a mesh count of not less than 20 mesh, more preferably not less than 30 mesh, preferably not more than 60 mesh, more preferably not more than 50 mesh.

The net-like structure also preferably has a mesh aperture of not more than 1 mm, more preferably not more than 0.8 mm, and even more preferably not more than 0.5 mm, preferably not less than 0.1 mm, more preferably not less than 0.2 mm.

The net-like structure also preferably has an opening ratio of not less than 20%, more preferably not less than 25%, and even more preferably not less than 30%, preferably not more than 65%, more preferably not more than 60%, and even more preferably not more than 55%.

Note that the "mesh count (unit: mesh) of a net-like structure" represents the number of openings per inch (25.4 mm), which may be calculated by, for example, the following expression (1).

$$\text{Mesh count (mesh)} = 25.4/(A + W) \qquad (1)$$

The "opening ratio (unit: %) of a net-like structure" may be calculated by, for example, the following expression (2).

$$\text{The opening ratio (\%)} = \{A/(A + W)\}^2 \times 100 \qquad (2)$$

(in the expressions (1) and (2), "A" represents a mesh aperture (mm), and "W" represents the wire diameter (mm) of the net-like structure)

In the cyclonic separation, the above wet cyclone may be used. When the cyclonic separation is employed in the second separation step, the discharge valve provided in the lower portion of the cyclone may be open or closed.

3. Returning Step

The present invention preferably comprises, a returning step of introducing the second purified liquid containing the easily polymerizable substance into the distillation column. The second purified liquid containing the easily polymerizable substance comprises the easily polymerizable substance and the polymerization inhibitor. Therefore, if the second purified liquid containing the easily polymerizable substance is introduced into the distillation column, the loss of the easily polymerizable substance may be reduced, and in addition, the cost of purification may be reduced. When the solid-liquid separation method in the second separation step is the gravitational sedimentation, a supernatant may be introduced into the distillation column. When the filtration is adopted, a filtrate after the filtration may be introduced into the distillation column.

The present application claims for benefit of priority based on Japanese Patent Application No. 2014-245161 filed on December 3, 2014.

EXAMPLES

The present invention will now be specifically described by way of examples.

Example 1

As the distillation column, a distillation column having an inside diameter of 2200 mm and including 50 stainless steel perforated trays without downcomer was used to carry out an operation of purifying a crude liquid containing acrylic acid. A column top part of the distillation column has an outlet of impurities having a low boiling point, and an inlet of reflux liquid (the inlet of reflux liquid is connected to an introducing pipe of reflux liquid). A column bottom part of the distillation column has an outlet of the bottoms liquid for discharging a bottoms liquid, and a returning inlet for returning a second purified liquid containing the easily polymerizable substance. An inlet of a treated liquid to which an introducing pipe for feeding the crude liquid containing the easily polymerizable substance is provided between the column top part and the column bottom part. A tank for temporarily storing the bottoms liquid, a liquid sending pump, and a wet cyclone were disposed to a pipe for the bottoms liquid. The distillation column was equipped with a reboiler (vertical multitubular type) where the liquid passes through the tubes in a natural circulation system. A liquid containing acrylic acid was extracted as a bottoms liquid from the column bottom part of the distillation column, and a flow rate of the bottoms liquid was controlled at 10,000 kg/h. Note that the bottoms liquid had a temperature of 100°C, and a density of about 1000 kg/m$^3$.

After temporarily stored in the tank, the bottoms liquid was transported to the wet cyclone ("Filstar (registered trademark)," manufactured by Industria Co., Ltd.) by the liquid sending pump. At this time, the speed of introducing the bottoms liquid into the wet cyclone was 0.98 m ·s$^{-1}$. A first purified liquid containing acrylic acid was obtained from an upper portion of the wet cyclone, and a liquid containing an insoluble solid such as a polymer was obtained from a lower portion of the wet cyclone. In this example, the opening degree of the valve provided at the lower exit of the wet cyclone was adjusted so that the liquid containing the insoluble solid was discharged at a rate of 300 kg/h. In this example, 100% of an insoluble solid having a grain diameter of not less than 450 μm comprised in the bottoms liquid was successfully collected into the liquid containing the insoluble solid by the wet cyclone. Note that the wet cyclone shown in FIG. 2A and FIG. 2B had the following dimensions.

- The pipe diameter "a" of the introduction part 2: 60 mm
- The pipe diameter "d" of the inlet/outlet part 3: 170mm; the length "h": 105 mm
- The shape of the hopper part 4: cylindrical; the pipe diameter "D": 95 mm; the length: 880 mm
- The pipe diameter "b" of the upper exit 6 of the discharge part 5: 60 mm
- The length "H" of the wet cyclone: 1124 mm

The liquid containing the insoluble solid discharged from the lower portion of the wet cyclone was transported to another storage tank. Acrylic acid was collected from the transported liquid containing the insoluble solid if necessary.

In such a situation, the operation was continued for 8000 hours yearly. During this period, the clogging did not occur in the wet cyclone, and it was not necessary to clean the wet cyclone. The total loss of acrylic acid during the 8000-h operation was about 24 tons.

Example 2

An operation was carried out in a manner similar to that of Example 1, except that the liquid containing the insoluble solid discharged from the wet cyclone was passed through a 40-mesh strainer to remove the insoluble solid, and a second purified liquid containing acrylic acid passed through the strainer was returned to the distillation column.

In such a situation, the operation was continued for 8000 hours yearly. During this period, the clogging did not occur in the wet cyclone, and it was not necessary to clean the wet cyclone. The strainer provided in a liquid sending line for the liquid containing the insoluble solid was cleaned 333 times/year. The total loss of acrylic acid during the 8000-h operation was about 2 tons.

Example 3

The strainer provided in the liquid sending line for the liquid containing the insoluble solid in Example 2 was replaced with a gravitational sedimentation tank having a volume of 50 L and a horizontally projected area of 0.07 m$^2$, and was adjusted so that a supernatant was returned to the distillation column. In the gravitational sedimentation tank, a second purified liquid containing acrylic acid was replaced at a frequency of once per two days.

In such a situation, the operation was continued for 8000 hours yearly. During this period, the clogging did not occur in the wet cyclone, and it was not necessary to clean the wet cyclone. The use of the gravitational sedimentation tank eliminated the necessity of cleaning of the gravitational sedimentation tank itself. The total loss of acrylic acid during the 8000-h operation was about 1.8 tons.

Example 4

An operation was carried out using equipment and conditions similar to those of Example 1, except that the quantity of the discharged liquid containing the insoluble solid was changed from 300 kg/h to 90 kg/h by adjusting the opening degree of the valve at the lower exit of the wet cyclone in Example 2. As a result, the clogging caused by a polymer was observed at a lower exit part of the wet cyclone about 2000, 4000, and 6000 hours (a total of three times) after the start of the operation. To remove the clogging caused by the polymer, the line was changed to a bypass, and the lower exit part of the wet cyclone was cleaned, and thereafter, the line was put back to the original position. The total loss of acrylic acid during the 8000-h operation was about 4.8 tons.

Comparative Example 1

An operation was performed using equipment similar to that of Example 1, except that a 40-mesh strainer was provided instead of the wet cyclone. The operation was performed for 8000 hours yearly. The strainer was cleaned 1024 times/year. The total loss of acrylic acid during the 8000-h operation was about 25 tons.

DESCRIPTION OF THE REFERENCE CHARACTERS

| 2 | introduction part |
|---|---|
| 3 | inlet/outlet part |
| 4 | hopper part |
| 5 | discharge part |
| 6 | upper exit |
| 8 | lower exit |
| 9 | valve |
| 21 | distillation column |
| 22 | outlet of impurities having a low boiling point |
| 23 | inlet of a reflux liquid |
| 24 | introducing pipe of a reflux liquid |
| 25 | inlet of a treated liquid |
| 26 | outlet of a bottoms liquid |
| 27 | returning inlet |
| 28 | liquid sending pump |
| 29 | wet cyclone |
| 30 | solid-liquid separation means |
| 31 | reboiler |
| 32 | condenser |

**Claims**

1. A method for purifying an easily polymerizable substance by distillation, comprising:

   a step of introducing a crude liquid containing the easily polymerizable substance into a distillation column,
   a step of discharging a bottoms liquid from a collection part of the distillation column, and
   a first separation step of introducing the bottoms liquid into a wet cyclone, to separate a first purified liquid containing the easily polymerizable substance from a liquid containing an insoluble solid,
   wherein the easily polymerizable substance is an unsaturated carboxylic acid or an ester thereof.

2. The method according to claim 1, wherein,
   the insoluble solid having a grain diameter of not less than 450 $\mu$m is removed by the wet cyclone, and
   a ratio of removing the insoluble solid having a grain diameter of not less than 450 $\mu$m is not less than 90%.

3. The method according to claim 1 or 2, wherein
   a speed of introducing the bottoms liquid into the wet cyclone is not less than 0.1 m·s$^{-1}$ and not more than 2.0 m·s$^{-1}$.

4. The method according to any one of claims 1 to 3, wherein
   the bottoms liquid introduced into the wet cyclone has a temperature of higher than 70°C and lower than 120°C.

5. The method according to any one of claims 1 to 4, wherein
   a flow rate per unit time of the liquid containing the insoluble solid is not more than 10% of that of the bottoms liquid.

6. The method according to any one of claims 1 to 5, further comprising:
   a second separation step of separating at least a portion of the insoluble solid comprised in the liquid containing the insoluble solid to obtain a second purified liquid containing the easily polymerizable substance.

7. The method according to claim 6, further comprising:
   a returning step of introducing the second purified liquid containing the easily polymerizable substance into the distillation column.

**8.** The method according to any one of claims 1 to 7, wherein
the easily polymerizable substance is acrylic acid, methacrylic acid, an acrylic acid ester, or a methacrylic acid ester.


**Patentansprüche**

**1.** Verfahren zur Reinigung einer leicht polymerisierbaren Substanz durch Destillation, umfassend:

einen Schritt des Einführens einer Rohflüssigkeit, die die leicht polymerisierbare Substanz enthält, in eine Destillationskolonne,
einen Schritt des Ausspeisens einer Bodenflüssigkeit aus einem Sammelteil der Destillationskolonne und
einen ersten Trennungsschritt des Einführens der Bodenflüssigkeit in einen Nass-Zyklon, um eine erste gereinigte Flüssigkeit, die die leicht polymerisierbare Substanz enthält, von einer Flüssigkeit zu trennen, die einen unlöslichen Feststoff enthält,
wobei die leicht polymerisierbare Substanz eine ungesättigte Carbonsäure oder ein Ester davon ist.

**2.** Verfahren nach Anspruch 1, wobei
der unlösliche Feststoff, der einen Korndurchmesser von nicht weniger als 450 $\mu$m aufweist, durch den Nass-Zyklon entfernt wird und
ein Anteil der Entfernung des unlöslichen Feststoffs mit einem Korndurchmesser von nicht weniger als 450 $\mu$m nicht weniger als 90% beträgt.

**3.** Verfahren nach Anspruch 1 oder 2, wobei
eine Geschwindigkeit des Einführens der Bodenflüssigkeit in den Nass-Zyklon nicht weniger als 0,1 m·s$^{-1}$ und nicht mehr als 2,0 m·s$^{-1}$ beträgt.

**4.** Verfahren nach einem der Ansprüche 1 bis 3, wobei
die in den Nass-Zyklon eingeführte Bodenflüssigkeit eine Temperatur von mehr als 70°C und weniger als 120°C aufweist.

**5.** Verfahren nach einem der Ansprüche 1 bis 4, wobei
eine Strömungsrate pro Zeiteinheit der Flüssigkeit, die den unlöslichen Feststoff enthält, nicht mehr als 10% derjenigen der Bodenflüssigkeit beträgt.

**6.** Verfahren nach einem der Ansprüche 1 bis 5, ferner umfassend:
einen zweiten Trennungsschritt des Abtrennens mindestens eines Teils des unlöslichen Feststoffs, der in der Flüssigkeit enthalten ist, die den unlöslichen Feststoff enthält, um eine zweite gereinigte Flüssigkeit zu erhalten, die die leicht polymerisierbare Substanz enthält.

**7.** Verfahren nach Anspruch 6, ferner umfassend:
einen Rückführschritt des Einführens der zweiten gereinigten Flüssigkeit, die die leicht polymerisierbare Substanz enthält, in die Destillationskolonne.

**8.** Verfahren nach einem der Ansprüche 1 bis 7, wobei
die leicht polymerisierbare Substanz Acrylsäure, Methacrylsäure, ein Acrylsäureester oder ein Methacrylsäureester ist.


**Revendications**

**1.** Procédé de purification d'une substance facilement polymérisable par distillation, comprenant :

une étape consistant à introduire un liquide brut contenant la substance facilement polymérisable dans une colonne de distillation,
une étape consistant à décharger un liquide de fond d'une partie collectrice de la colonne de distillation, et
une première étape de séparation consistant à introduire le liquide de fond dans un hydrocyclone, pour séparer un premier liquide purifié contenant la substance facilement polymérisable d'un liquide contenant un solide insoluble,

où la substance facilement polymérisable est un acide carboxylique insaturé ou un ester de celui-ci.

2. Procédé selon la revendication 1, où
le solide insoluble ayant un diamètre de grain d'au moins 450 μm est éliminé par l'hydrocyclone, et
un rapport d'élimination du solide insoluble ayant un diamètre de grain d'au moins 450 μm est d'au moins 90 %.

3. Procédé selon la revendication 1 ou 2, où
une vitesse d'introduction du liquide de fond dans l'hydrocyclone n'est pas inférieure à 0,1 m·s$^{-1}$ et pas supérieure à 2,0 m·s$^{-1}$.

4. Procédé selon l'une des revendications 1 à 3, où
le liquide de fond introduit dans l'hydrocyclone a une température supérieure à 70° C et inférieure à 120° C.

5. Procédé selon l'une des revendications 1 à 4, où
un débit par unité de temps du liquide contenant le solide insoluble n'est pas supérieur à 10% de celui du liquide de fond.

6. Procédé selon l'une des revendications 1 à 5, comprenant en outre :
une seconde étape de séparation consistant à séparer au moins une partie du solide insoluble compris dans le liquide contenant le solide insoluble pour obtenir un second liquide purifié contenant la substance facilement polymérisable.

7. Procédé selon la revendication 6, comprenant en outre :
une étape de retour consistant à introduire le second liquide purifié contenant la substance facilement polymérisable dans la colonne de distillation.

8. Procédé selon l'une des revendications 1 à 7, où
la substance facilement polymérisable est l'acide acrylique, l'acide méthacrylique, un ester d'acide acrylique ou un ester d'acide méthacrylique.

[Fig.1]

Crude liquid
containing the easily
polymerizable
substance

Distillate

First purified liquid containing
the easily polymerizable
substance

Residue

[Fig.2A]

First purified liquid containing the easily
polymerizable substance

Liquid containing an insoluble solid

[Fig.2B]

First purified liquid containing the easily
polymerizable substance

Liquid containing an insoluble solid

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2001030120 A
- US 2002008064 A
- JP 2002001017 A
- JP 2001293301 A
- JP 2014245161 A